# EUROPEAN PATENT APPLICATION

(11) **EP 4 814 096 A1**
(43) Date of publication of application: **30.09.2026**
(21) Application number: 25166078.3
(22) Date of filing: 25.03.2025
(51) Int. Cl.: C12M 1/00, C12M 1/40, C12M 1/26

(54) **METHOD FOR SELECTIVE BIOCATALYST SEPARATION IN HIGH PRODUCING BIOCATALYTIC REACTOR SYSTEMS**

(71) Applicant: Delft Advanced Biofuels B.V., 2613 AX Delft (NL)
(72) Inventor: NELISSE, Pieter Marinus, 2613 AX Delft (NL); VAN LOTRINGEN, Marion, 2613 AX Delft (NL)
(74) Representative: V.O.

(57) **Abstract**

Method for selective cell retention of a micro-organism comprising a (semi-)continuous fermentative production of an organic substance of interest, in a bioreactor system, where fluidly connected is present
(a) a fermentation zone, wherein the organic substance is produced using a biocatalytic micro-organism, and
(b) a separation zone, wherein a reaction mixture, comprising an aqueous fraction, containing the biocatalytic micro-organism, and the produced substance, is fed to the separation zone, or wherein at least the aqueous fraction containing the biocatalytic microbiological cells is fed to the separation zone, and wherein the reaction mixture is separated in two fraction, distinguished in solid content, as in the separation zone, the first liquid fraction is enriched in the biocatalytic micro-organism (relative to the second liquid fraction) and the second liquid fraction is enriched in a solid matter different from the biocatalytic micro-organism (relative to the first liquid fraction).

## Description

The present disclosure relates to a method for fermentatively producing an organic substance. In particular, the present disclosure relates to a method, comprising a selective cell retention of a micro-organism used as a biocatalyst during a continuous or semi-continuous fermentative production of an organic substance.

There is a strong world-wide effort to provide more sustainable production routes, e.g. transition from fossil based substances to bio-based substances, for production of various organic substances, e.g. alcohols, esters, amino acids, carbohydrates, ethers, lipids, ketones, aldehydes, organic acids, pyridines, and proteins. These substances can be used in a variety of applications for example as flavours, fragrances, cosmetic ingredients, food ingredients, nutraceutical products, bioinsecticides, specialty chemicals, commodity chemicals, or as biofuels. These substances can either provide a sustainable replacement of a known product or a novel more sustainable product.

These more sustainable production routes include amongst others biocatalytic processes. It is known in the art to produce organic substances by biocatalytic processes such as fermentation and cell-free conversions. In a fermentation process, micro-organisms are used to convert a suitable substrate into an organic substance of interest. In a cell-free conversion, enzymes convert a substrate into an organic substance of interest. As is generally known in the art, various micro-organisms and enzymes are known that can be used on an industrial scale for the production of a wide variety of organic substances. Generally known fermentative processes include the use of natural micro-organisms and/or genetically modified organisms, e.g. yeasts or bacteria, for the production of various organic substances, e.g. alcohols, esters, amino acids, carbohydrates, lipids, ketones, aldehydes, organic acids, ethers, pyridines, imines, proteins.

Organisms may be genetically modified to increase the product titre of a naturally produced organic substance and/or to enable a microorganism to produce an organic substance that it does not produce naturally. E.g. a micro-organism may be modified by incorporating genes from a plant responsible for the production of an organic substance, e.g. a terpene, a terpenoid, not naturally produced by the micro-organism.

Current high producing continuous or semi-continuous fermentative biocatalytic reactor systems, most often chemostat, lack the ability for selective separation of biocatalytic micro-organisms (biocatalysts) from other solid matter. This lack of selective biocatalyst separation results in the following potential debits. (1) In any system, but especially for higher biocatalyst retention systems like air lift reactors and membrane bioreactors, (aged) biocatalyst and/or biocatalyst material with reduced to no activity builds up in the biocatalytic reaction medium leading to reduced biocatalytic reaction activity and efficiency as the run progresses. (2) In case of different populations of biocatalyst present inside the biocatalytic reaction system there is currently no selective way to control the size of each population other than trying to apply selection pressures onto the system to influence the population sizes. (3) In case of a solid organic product that precipitates in the biocatalytic medium there is currently no selective means of separation of the product from the biocatalytic micro-organisms and/or other microbial matter.

Especially in the waste water treatment industry (WWTP) more extensive research has been done into biocatalyst retention and population shifts in continuous flow systems. However in Waste water treatment the systems are focussed on converting matter and removing organic content and nitrogen, by activated sludge based systems. The reactor systems are not used for the production of end-products and are also mixed-culture (open) systems. Due to the diluted (feed) nature of the system, as water treatment is the objective, the liquid flowrates, and the corresponding hydraulic retention time of the system differs. Many reactor technologies and their impact on solid retention time (SRT) in combination with hydraulic retention time (HRT) and a specific conversion of components have been investigated. Reactor technologies tested comprise 'closed digester tank' (CDT, ), 'Continuously stirrer tank reactor' (CSTR,), Anaerobic sequencing batch reactor, stirred tank, modified 'Anaerobic baffled reactor' (ABR), 'upflow anaerobic sludge blanket' (UASB,), 'Expanded granular sludge blanket' (EGSB), 'upflow anaerobic sludge fixed film reactor' (UASFF), 'Anaerobic fluidized bed' (AFB), 'membrane bioreactor' (MBR), Anaerobic filter. These reactor systems operate at relatively low growth rates (mu) in the range of 0.023 h⁻¹ to 0.11 day⁻¹, lower feed substrate concentrations than typical biocatalytic reaction systems (~0.3 to 19 wt%), relative low biocatalyst concentrations 'mixed liquor suspended solids' (MLSS) of 1.5-5 g/L for stirred tank kind of systems with similar SRT to HRT, but for airlift reactor systems as well and an MLSS of 10-12 g/L for systems with higher SRT (like a MBR) and relatively lower substrate conversion rates (~0.02 to 0.66 g/L/h) than typical biocatalytic reaction systems. Typical SRT ranged from 0.8 to 100 days and the HRT ranged from 0.3 to 16 days. It was found in this research that there is an optimum and even higher SRT isn't always better. In all this is shown to illustrate that the application of solid and liquid residence time in WWTP covers a wide range and cannot be used to optimize a specific biocatalytic conversion step without undue burden.

Especially relevant for assessment of more intensified systems in WWTP, the 'Anammox' processes is relevant; Also here solid retention time (and population distribution) control is desired but is limited, as shown by [Lackner et al, Full-scale partial nitridation/anammox experiences An application survey, water research 55 (2014) 292-303] and they surveyed authors stating: "Aside from growing and sustaining the slow growing anammox bacteria, balanced activity of aerobic ammonium oxidizing bacteria needs to be established in line with a suppression or out-selection of nitrite oxidizing bacteria. The growth rates of ammonium oxidizing bacteria are usually higher than those of nitrite oxidizing bacteria at elevated temperatures (> 30°C) which makes selective wash-out of nitrite oxidizing bacteria possible in suspended biomass systems for partial nitridation by adjusting the solids retention time (SRT) at a minimum level That concept, however, is not applicable for biofilm systems because biofilms can sustain microorganisms with very different growth kinetics due to the undefined SRT and their distinct substrate gradients; further also for combined PN/A SRT cannot be applied as sole selection criterion due to the significantly slower growth rate of the anammox biomass. The most practical approach to limit nitrite oxidation is considered to be reactor operation under oxygen limited conditions which favours growth of ammonium oxidizing bacteria versus nitrite oxidizing bacteria, as their oxygen affinity is higher and combined with additionally competition for nitrite by the anammox bacteria."

Nicolella et al [wastewater treatment with particulate biofilm reactors, Journal of Biotechnology 80 (2000) 1-33] showed in Figure 2 that for Waste Water Treatment Systems, different reactor geometry and configurations, with their own intrinsic cell settling and retention properties, allow operation of waste water treatment, for a wide range of substrate concentrations and flowrates. However, high substrate concentrations in waste water treatment indicates the use of single cells (under high growth rate), as any dilution rate of the system is acceptable because wash-out is not limiting the operation in waste water treatment. For a fermentative production system this would lead to an undesirable product yield as substantial amount of substrate go to biomass growth.

Apart from certain reactor configurations, current methods for increased biocatalyst retention include immobilization the biocatalyst by fixating microbes or cell-free enzymes by for example a matrix, biofilm support structure, or pre-formed carrier. This does generally not allow selective separation of biocatalytically (highly) active living microbes from non-viable microbes that have at least substantially lost their activity. Further, fixation has drawbacks, such as the added complexity by having to immobilise the microbes, mass transfer limitation and a reduced biocatalytic activity, once fixated. Further, the support occupies inert space in the bioreactor system, which may also have an adverse effect on production capacity. Other methods include retention means of a membrane, which is generally not adequate either for selective retention of living cells relative to other solid matter. The use of membranes also adds to complexity plus brings typical challenges like risk of fouling of the membrane surface. Further, it has been proposed to separate biocatalyst from a liquid product by enhancing gravity of the biocatalyst itself by for example flocculation, like for example yeast flocculation for ethanol production in an ultra-low aerated biocatalytic reactor system (0.005 vvm aeration, (Zhao et al, Yeast flocculation New story in fuel ethanol production, Biotechnology Advances 27 (2009) 849-856). Retention of the flocs is problematic due to the CO2 formation of the high producing system, in relation to the requirement of effluent withdrawal under the given conditions. For aerobic systems an even higher gas input is required, due to oxygen now being required as substrate.

Traditionally fermentations were carried out using a batch process, but nowadays continuous and/or semi continuous fermentative processes are also known in the art. A (semi-)continuous operation allows for control of residence time of certain fluid phases and/or substances in the system. In combination with continuous in-situ substance recovery, process sustainability and economic viability can be improved. WO2021/010822 and WO2024/049295 disclose devices and methods allowing the continuous fermentative production and extraction of produced product in a single apparatus, using extractive overlay fermentation. Herein liquid-liquid phase separation by gravitational forces is applied as In Situ Product Recovery power (ISPR), which is integrated in the fermentation reactor system itself.

Although the devices and methods of WO2021/010822 and WO2024/049295 thus allow for an additional degree of freedom in operation and allows matching of ISPR and separation capacity or requirement, it does not describe how to selectively separate a desired active micro-organism, used as a biocatalyst for the production of the organic substance of interest, directly from other solid matter, like biocatalyst material with reduced to no activity, a different cell phenotype, other species of cells ('contamination) and precipitated product in the bioreactor system.

It is an object of the present disclosure to provide a novel method wherein an organic substance is fermentatively produced, in particular a method that addresses one or more drawbacks of known methods to produce an organic substance with the help of a biocatalyst, for instance one or more drawbacks known or mentioned for prior art methodology, as described herein.

The inventors realised in particular that current technologies suffer from limitations like declining biocatalyst performance over time, reduced mass transfer, restrictions on other process operating parameters like aeration, high material cost, emulsion formation in the fermentation broth over time, fouling, pressure drop and the risk of contamination (introduced by external equipment for biocatalyst rich recycle streams like centrifuges).

The inventors realised that it is possible to selectively retain biocatalytically active microbiological cells used for fermentative preparation of an organic substance of interest in a method comprising a (semi-)continuous operation of a bioreactor system. Moreover, they realised that this can be achieved by selectively separating biocatalytically active micro-organisms from other solid matter in a (semi-)continuously operated biocatalytic reactor system, and that this can be used to contribute to maintaining a satisfactory or even improved substrate conversion rate to a product of interest for prolonged run duration.

Amongst others, the inventors realised that to enable optimisation on yield for fermentative productive systems more specific biomass retention and separation is required. Overall the flowrate of the substrate feed in, the substrate concentration and yield together give the productivity of the system. Product retention time is also a design parameter in production systems. This reinforces that the growth rate, the degree of cell retention (growth requirement) and ability to retain viable cell matter is an important requirement for reactor and process design. When combined more effectively this enables the development of processes with increased performance, specifically yield.

Accordingly the present disclosure relates to a method comprising a (semi-)continuous fermentative production of an organic substance of interest, in a bioreactor system comprising
(a) a fermentation zone, wherein the organic substance is fermentatively produced using a biocatalytic micro-organism, and
(b) a separation zone, wherein a reaction mixture, said reaction mixture comprising an aqueous fraction, containing the biocatalytic micro-organism, and the produced organic product, is (semi-)continuously fed to the separation zone, or wherein at least the aqueous fraction containing the biocatalytic microbiological cells is (semi-) continuously fed to the separation zone, and wherein the reaction mixture is separated into at least two fractions, distinguished in solid content, as in the separation zone, the first liquid fraction is enriched in the biocatalytic micro-organism (relative to the second liquid fraction) and the second liquid fraction is enriched in a solid matter different from the biocatalytic micro-organism (relative to the first liquid fraction).

Thus the present method allows the selective retention of the biocatalytically active cells in the bioreaction system. The retained cells (present in the first fraction) or a substantial part thereof, are typically returned to the fermentation zone.

Typically, the method according to the present disclosure comprises a continuous or semi-continuous fermentative production of one or more organic substances in the bioreactor system in which conversion of substrate by the living micro-organism into at least one organic substance occurs in the fermentation zone, wherein the micro-organism is mobile in the reaction mixture. Thus, the biocatalytic micro-organism is (freely) distributed in the reaction mixture in the fermentation zone, at least during normal operation. The micro-organism is typically unbound to a support/carrier material. When freely distributed, typically at least a substantial part of the micro-organism is dispersed as individual cells, although it is also possible that biocatalytic cells form clusters essentially consisting of cells, in particular if the separation comprises some form of cluster formation, the clusters are returned to the fermentation zone, and the fermentation zone is operated under conditions at which the clusters are not fully disintegrated (such as when a relatively low stirring force is applies/relatedly low turbulence) and the microbes are actively pushed to aggregate to form solid particulates in the desired size range due to the applied conditions.

The reaction mixture is provided in the fermentation zone, which reaction mixture comprises the micro-organism and an aqueous phase, the aqueous phase comprising a substrate for the micro-organism. A substrate as such can be a liquid, a solid or a gas. A substrate is usually dissolved in the aqueous phase for efficient uptake by the micro-organism.

The organic substance is fermentatively prepared from the substrate in the presence of the micro-organism in the fermentation zone. The micro-organism thus acts as a biocatalyst.

A method according to the disclosure comprises a stage during which the fermentative production and a separation are simultaneously carried out, which stage may herein also be referred as the 'simultaneous production and separation stage', or 'simultaneous stage'. During at least a substantial part of said simultaneous stage, process conditions are usually in an essentially steady state, in particular when operating under continuous process conditions.

In accordance with a method of the present disclosure, the substrate is typically at least during a substantial part of said simultaneous production and separation stage, preferably during an essentially steady state production stage, continuously or semi-continuously fed into the fermentation zone.

In accordance with a method of the present disclosure, the reaction mixture, comprising the micro-organism and the produced organic substance, or at least an aqueous fraction of the reaction mixture, comprising the micro-organism and optionally the produced organic substance, is typically at least during a substantial part of said simultaneous production and separation stage, preferably during an essentially steady state production stage, continuously or semi-continuously fed from the fermentation zone into the separation zone.

In a method according to the disclosure, the reaction mixture, comprising the micro-organism and the produced organic substance, or at least an aqueous fraction of the reaction mixture, said aqueous fraction comprising the micro-organism, fed into the separation zone, is subjected to a separation into at least two fractions, thereby forming a first fraction enriched in the micro-organism and a second fraction enriched in solid matter different from said micro-organism in which the first fraction is enriched. The separation typically comprises the formation of at least two liquid layers (an upper layer and a lower layer), wherein one of said two layers forms the first fraction (enriched in the biocatalytic micro-organism) and the other of said two layers forms the second fraction (enriched in a different solid matter).

At least during said simultaneous stage, in particular during an essentially steady state, at least a part of said second fraction from the bioreactor system is typically continuously or semi-continuously removed from the bioreactor system. The removed (part of the) second fraction may be further processed by any suitable, e.g. known, downstream processing e.g. as described in the prior art described herein. Such downstream processing can comprise (semi)continuous processing, batch processing or both.

At least during said simultaneous stage, in particular during an essentially steady state, at least a part of said first fraction enriched in the micro-organism is returned from the separation zone to the fermentation zone.

The present disclosure thus advantageously provides a method for selective microbial biomass separation in biocatalytic reactor systems in particular a method for selective microbial biomass separation in (semi-)continuous high producing fermentation system that overcomes one or more of the disadvantages of known microbial retention systems and methods. Advantageously the method according to the present disclosure allows for maintaining substrate conversion rates for prolonged run duration, by desired fraction of biomass, those with i.e. preferably the optimal conversion rate and product yield. This is especially beneficial for fermentation processes with slow growth. This method can be applied to both aerobic and anaerobic processes, in both general and precision fermentation processes.

A method according to the present disclosure can be applied to any fermentation; it has specific benefits for processes that suffer from one or more of: product instability, product inhibition, an equilibrium limitation, a product yield limitation due to excess biomass formation, biocatalytic biomass activity loss over time.

A major benefit of the present method is to enable or facilitate desired prolonged microbial activity (longer effective fermentation time), in particular enabling or facilitating maintaining substrate conversion rates for a prolonged run duration.

The method of the present disclosure allows for process improvements like improved yield, reduced downtime, reduced resource requirements, reduced energy consumption. Leading to improved sustainability and improved economic feasibility of the process and therefore according to the present disclosure thus enables more fermentation processes to be competitive and successful in the current market.

In particular effective is a method according to any of the claims 1-14.

The method of the present disclosure can be beneficial for the upstream processing (biocatalytic reaction) as well as the downstream processing and/or purification.

The disclosure mitigates one or more restrictions of known methods, like declining microbial performance over time, reduced mass transfer, restrictions on other process operating parameters like aeration, high material cost, fouling, pressure drop and the risk of contamination (introduced by external equipment for biocatalyst rich recycle streams like centrifuges.

The selective retention of the biocatalytic micro-organism can be used to stimulate selection pressure for a slow-growing biocatalytic micro-organism with a favourable production rate or yield. This can be accomplished by biocatalytic micro-organism retention in the fermentation zone (via returning said micro-organism in the first phase to the fermentation zone) in combination with removal of biomass with reduced or no activity towards the fermentative, or by population selection due to selective separation and removal of faster growing biocatalytic micro-organisms.

Terminology used for describing particular embodiments is not intended to be limiting of the invention. Unless defined otherwise herein, terms are as defined in WO2021/010822 and WO2024/049295. When not defined the terms are used in agreement with how they generally defined in the art.

As used herein, the singular forms "a", "an" and "the" are intended to include the plural forms as well, unless the context clearly indicates otherwise. The term "or" as used herein is defined as "and/or" unless specified otherwise.

The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The term "(at least) substantial(ly)" or "essentially" is generally used herein to indicate that it has the general character or function of that which is specified. When referring to a quantifiable feature, this term is in particular used to indicate that it is at least 50 %, more in particular more than 75 %, even more in particular more than 90 % of the maximum that feature. The term 'essentially free' is generally used herein to indicate that a substance is not present (below the detection limit achievable with analytical technology as available on the effective filing date) or present in such a low amount that it does not significantly affect the property of the product that is essentially free of said substance. In practice, in quantitative terms, a product is usually considered essentially free of a substance, if the content of the substance is 0 - 1 wt.%, in particular 0 - 0.5 wt.%, more in particular 0 - 0.1 wt.%.

In the context of this application, the term "about" means generally a deviation of 10 % or less from the given value, in particular a deviation of 5% or less, more in particular a deviation of 2% or less.

As used herein "organic" refers to any organic substance (such as biocatalytically produced product that is chemically oxidisable, as can be determined by the Chemical Oxygen Demand (COD) test, as described in ISO 6060:1989.

Terms like 'biocatalytical(ly)' and " biocatalyst" are generally known in the art to describe methods respectively biological material, wherein at least one reaction step in the method is catalysed by a biological material or moiety derived from a biological source, for instance an organism or a biomolecule derived there from. In the method of the present disclosure an organic substance is fermentatively produced, i.e. the biocatalyst is a living micro-organism. The micro-organism generally excretes the produced organic substance of interest into the reaction medium from which the organic substance can be recovered without having to lyse the micro-organism. Thus a method of the present disclosure is generally carried out without having to disrupt the biocatalyst in order to recover the product. Fermentative methods can be aerobic, oxygen-limited or anaerobic.

For the purpose of clarity and a concise description, features are described herein as part of the same or separate embodiments, however, it will be appreciated that the scope of the invention may include embodiments having combinations of all or some of the features described.

The operation of the fermentation zone, including the feeding, choice of micro-organism and substrate can be based on known methods, e.g. as described in the prior art cited herein, notably WO2021/010822 or WO2024/049295 of which the contents are incorporated by reference.

At least during an essentially steady state stage, the fermentation zone is typically operated under turbulent flow conditions, whilst the separation into said first fraction and said second fraction takes place under less turbulent or laminar conditions. The selective separation, resulting in said first fraction enriched in the biocatalytic micro-organism is enhanced by changing hydrodynamic conditions inside the biocatalytic reactor system. In particular a more laminar flow, resulting in more calm flow conditions enhances solid-solid, solid-liquid, liquid-liquid, gas-liquid separation locally in the reactor system. The Reynolds number (Re), is a generally known dimensionless number defined as the ratio of inertial forces to viscous force (Re = density x fluid velocity x characteristic length / dynamic viscosity ), that is used in the art to quantify the flow conditions. A low Re, typically of about 2300 or less is indicative of laminar flow, preferably of 2000 or less, e.g. 100-1800. A high Re, typically of about 2900 or more, e.g. 3500-10000, is indicative of turbulent flow. However, a person skilled in chemical engineering will also be able to determine visually whether flow conditions are essentially laminar (e.g. by injecting some ink and watching the flow pattern). Thus, the flow, at least in a part of the separation zone, used for settling of solid matter, usually has a Reynolds number (Re) of less than 3000, preferably of 2900 or less, more preferably of less than 2300. It is not necessary that there is a continuous flow though. Essentially laminar flow conditions and no-flow conditions can be alternated in the reaction zone.

The changing of hydrodynamic flow conditions allows for increased relative movement of a certain phase, particle and/or droplet up and an increased relative movement of another certain phase, particle and/or droplet downwards. In case of large enough particles and/or droplets this phenomena takes place to a significant enough extent to allow for sufficient separation. In case there is a need for even further enhanced separation, a second liquid phase may be used and/or other operating conditions may be changed locally to further enhance separation. This second liquid phase is of particular benefit in case of smaller hydrophobic, a-polar material, like cell debris, precipitated product, extracellular polymeric substance (EPS), etc.. Thus, changing hydrodynamic flow conditions is of particular benefit in the case of using a difference in size between the solid matter for the first fraction (comprising the micro-organism to be retained) and the solid matter for the second fraction and/or droplet size of a liquid phase comprising fermentatively produced organic substance (e.g. when the method of the present disclosure is a method comprising an in-situ organic substance extraction with a liquid extraction phase, for instance in accordance with WO2021/010822 or WO2024/049295).

The separation into said first fraction and said second fraction generally makes use of at least one difference in a physical property between the micro-organism for the first fraction and the solid matter for the second fraction. Advantageously, at least one physical property is used selected from the group of specific gravity (relative density), electrostatic forces, polarity/non-polarity, size, shape, magnetic properties and hydrophobicity. Carrying out such separation is, e.g., beneficial for yield of the produced organic substance of interest.

In preferred embodiment the degree of hydrophobicity of a solid matter for the first fraction or the second fraction is influenced by changing one or more operating parameters, in particular one or more of pH, temperature, presence of ionic species in the biocatalytic aqueous liquid phase, changing the state of the product itself (e.g. in case of acids/bases change from dissociated to non-dissociated form/ change from acid to its conjugated base or change from base to its conjugated acid), the cell membrane, folding state of protein (as a produced organic substance), aggregation state, material selection, charge state of the molecule itself, polarity of the molecule or group of molecules.

In an advantageous embodiment, use is made of a difference in size, resulting in a difference in gravitational, buoyancy and/or drag forces, allowing for selective separation by settling.

In an advantageous embodiment, the difference in the physical property, more specifically size, is enhanced by forming enlarged biomass particulates from the micro-organism used for the microbial conversion, e.g. by creating clusters of micro-organism cells, which forming comprises at least one of (1) aggregation, (2) flocculation, and (3) filamentation, and wherein said first fraction comprises said enlarged biomass particulates. In a further advantageous embodiment, at least one of aggregation, flocculation and filamentation is used to create (enlarged) particular of the solid matter for the second fraction. Such means of forming clusters are known in the art per se. The skilled person will be able to realise this in a method according to the present disclosure based on the present disclosure, the cited prior art and common general knowledge.

The difference in the physical property, more specifically size, is enhanced by forming enlarged biomass particulates from the micro-organism used for the microbial conversion, e.g. by creating clusters of micro-organism cells, which forming comprises at least one of aggregation, flocculation, and filamentation, and wherein said first fraction comprises said enlarged biomass particulates. Dependent on the used biocatalytic micro-organism (or the nature of an undesired micro-organism of which the removal via the second fraction is to be accomplished) aggregation, flocculation, or filamentation can be naturally occurring and/or influenced by setting certain operating conditions optionally in combination with a certain reactor geometry (flow conditions, shear forces). The net sum will dictate the form and size of the flocs, aggregates or particles of filamented cells.

Auto-flocculation or auto-aggregation can be influenced at biocatalyst level through microbe choice and selection, modifications and aggregate properties at cell level such as the surface properties and ability of cells to bind to each other, mass transfer properties, stability of the three-dimensional complex amongst other properties. Next to biocatalyst selection and modifications auto-flocculation or auto-aggregation can be influenced by process conditions, like amount of shear, power input, stirrer tip speed, flow conditions and velocities, presence of particulates. Flocculation can also be accomplished by addition of a flocculant. Various flocculation agents have been employed for the immobilisation or flocculation of biomass including inorganic, organic and microbial flocculants. Organic flocculants are often advantageous as they are fast acting and often do not require large dosages. Examples known in the art of organic flocculants include chitosan, polyacrylamide, polyethylene amine. Inorganic flocculants include salts of aluminium such as poly-aluminium chloride and aluminium sulphate as well as salts of iron including ferric chloride and ferric sulphate. Although salts of aluminium, iron and zinc can provide efficient flocculation of microalgae, these salts would often end up in the natural environment, causing detrimental environmental effects via soil and water pollution, or in the biomass, which may impact or limit applications.

A cationic flocculant maybe used to attract negatively charged particles in the water, such as certain types of dirt, organic materials, or microorganisms. Chitosan is an example of an organic cationic flocculant. Calcium ions, aluminium ions and iron ions are examples of inorganic cationic flocculants. A flocculant can also be anionic, attracting particles with a positive charge. The choice of a cationic or anionic flocculant depends on the aqueous environment conditions, like alkalinity in combination with the electrical properties of the solids to be flocculated.

A potential disadvantage associated with the use of natural or chemical flocculation for immobilisation is the relative weak strength of the microbial clusters and their fragmentation when exposed to shear stresses. Shear stresses are imposed through hydrodynamic forces in biocatalytic reactors by impeller mixing in the bulk fluid phase or fluid motions associated with the gas-liquid interface if gas-sparging is used. These shear stresses should be minimized to prevent fragmentation of the microbial flocs through weaker stirring or lower gas flow rates, possibly at the expense of decreased mass transfer rates and overall biocatalytic reactor performance, if the micro-organism is used in flocculated state in the fermentation zone.'

Carriers for a biocatalyst often create large difference in retention and thus limit the type and geometry of the reactor applicable for an applicable retention strategies. Thus it is a major advantage that the present method allows the selective retention of non-supported biocatalytic micro-organisms.

Microbes exists that can form filaments, thereby enlarging themselves. This can also be used to enhance selective separation in accordance with the disclosure, in particular when a filament forming micro-organism is used for the fermentative production. A well-known class that does this is the class of filamentous fungi. Typical for filamentous fungi is a high biomass density (~5-100 g/L), high titres, eukaryotic 'post-translational modifications' (PTM) and longer fermentation times. However the filamentous nature introduces some challenges as well, like increased broth viscosity. Preferred examples of filamentous fungi include Aspergillus, in particular *A. niger, A. flavus, A terreus; Rhizopus arrhizus; Fusarium oxysporum*; and *Trichoderma reesei.*

In a preferred embodiment the selective separation is enhanced by using a second, liquid phase, as an auxiliary liquid. In a first preferred embodiment the auxiliary liquid is an organic liquid, forming a separate liquid phase. It is found that more hydrophobic, apolar material (than the microbiological cells) can be enriched at the interface between the two liquid phases, i.e. the aqueous phase of the reaction mixture and the auxiliary liquid phase. By selectively separating the auxiliary liquid phase from the aqueous phase the more hydrophobic, a-polar material is transported with the second auxiliary phase, as (part of) the second fraction, and selectively removed from the biocatalytic reactor system. In a further preferred embodiment, the auxiliary liquid forms a second aqueous phase, separate from the first aqueous fraction. This can be accomplished by forming emulsions/dispersions of the water-in-water type, in the presence of suitable aids, which can be polymeric or a salt. Advantageously, said second liquid phase comprising an aqueous phase with either a polymer and a salt or with two or more polymers. When using at least two or more polymers, these are chosen to be incompatible with each other, whilst individually dissolved in the aqueous phase. Thus at least two aqueous phases form. Suitable incompatible water-soluble polymer combinations are known in the art. Exemplary mutually incompatible polymers systems for water-in-water type emulsions are polyethylene glycols (PEG) and dextrans. An example of a combination of a salt and a polymer is PEG and potassium phosphate.

Further, in an advantageous embodiment, the separation into said first and said second fraction is aided by the presence of a gas phase, said gas phase either being locally introduced and/or being the result of gas entraining in the aqueous fraction to the separation zone as result of hydrodynamics. This is in particular useful when employing flotation.

In a preferred method of the present disclosure, the enrichment in the first fraction with biocatalytic micro-organism comprises the use of settling. In such method, the method is advantageously operated to provide an observed settling rate of 0.002 cm/min to 1.4 cm/min of particles of the micro-organism cells (such as aggregates, flocs, filamented micro-organism). This typically corresponding to a (spherical) particle size of about 7 micron to about 0.5 mm). More preferably, said particles have an observed settling rate of 0.01 cm/min to 0.8 cm/min (typically corresponding to a size of about 10 micron to about 0.3 mm). In a specific embodiment, said particles have an observed settling rate of 0.08 cm/min to 0.5 cm/min (typically corresponding to a size of about 50 micron to about 0.15 mm). The skilled person will be able to choose method conditions to effectuate such settling rate based on the information provided in the present disclosure, the cited prior art, and common general knowledge.

In a preferred embodiment the selective separation into the first and second fraction is enhanced by the biocatalytic reactor geometry design, in combination with the degree of freedom and control of the process resulting from the selected reactor geometry. As is known in the field settling or sedimentation can be aided by angle of the reactor internal or wall, particular lamella type settling at 45°-70°, often 60° angle is typically applied. Reactor geometry can affect separation by influencing flow conditions, altering an angle in a channel between fermentation zone and separation zone (e.g. when using a system based on WO2021/010822), location and number of outlets for the second fraction. E.g. use can be made of a principle described in WO2024/049295 in combination with the contents of the present disclosure. Geometries like slopes angles to enhance e.g. degassing or optimize settling are known in the art and can be readily applied [e.g.: Zhao et al, Yeast flocculation New story in fuel ethanol production, Biotechnology Advances 27 (2009) 849-856; Domingues et al. Contamination of a High -Cell-Density Continuous Bioreactor, Biotechnol. Bioeng. 68 (2000) 584-587; Zhang et al, A New Developed Airlift Reactor Integrated Settling Process and Its Application for Simultaneous Nitrification and Denitrification Nitrogen Removal, The Scientific World Journal (2013), p1-7].

Advantageously, in the method of the present disclosure,
- the retention time of said first fraction is significantly larger than the hydraulic retention time of the (main) aqueous phase in the bioreactor system;
- the retention time of said second fraction is equally or lower than the hydraulic retention time of the (main) aqueous phase in the bioreactor system;
- further, in case the separation is aided by the presence of a second liquid and/or gas phase the retention time of said second fraction is lower than the hydraulic retention time of the (main) aqueous phase in the bioreactor system.

The produced organic substance of interest can be recovered from the bioreactor system as part of the second fraction or a third fraction. Besides using the present method to separate the organic substance as a solid, it is also possible to use (semi-continuous) in situ extraction, e.g. essentially as described in WO2021/010822 or WO2024/049295. When using in situ extraction, in case of a hydrophobic produced organic substance it can form its own phase. It is also possible to include a separate extraction phase (recovery liquid), which can be organic or inorganic. It is also possible to make use of the water-in-water emulsion principle, wherein one of the aqueous phases serves as a product recovery phase.

A method according to the present disclosure allows fermentative production and recovery of organic substances for a prolonged time at a high liquid capacity for various types of product-recovery combinations, including for 1) organic substances that form their own liquid phase; 2) organic substances that are produced and extracted in situ (e.g. 2-phenyl ethanol (2-PE), butanol), using an extractant (product recovery phase); 3) product in aqueous phase of the reaction mixture (e.g. proteins). For illustrative purposes, the following table lists a typical product formation rate (g/L/h) and substrate yield (g/g) for exemplary products in each of these cases. The product retention time (PRT) follows from the liquid (or product recovery phase) retention time. The substrate used are concentrated (glucose) feed, 50 weight % solutions, unless specified otherwise.

| Case | Product formation rate [g/l/h] | Substrate yield [g/g] | PRT [h] |
|---|---|---|---|
| 1 self-formed liquid phase | 2.5 | 0.25 | 6 |
| | 5 | 0.25 | 3 |
| 2 extraction (2-PE) | 1 | 0.1 | 1 |
| 2 extraction (butanol) | 5 | 0.3* | 1-2 |
| 3 product in aqueous phase (protein)^{#} | 1 | 0.1 | 100^{@} |

| | | | |
|---|---|---|---|
| *70 wt% # non-selective recovery via aqueous phase (reaction mixture) @ equal to HRT | | | |

The PRT is high in this example for case 3 (protein recovery in aqueous phase). The PRT (=HRT) can be reduced by diluting the feed, which requires more specific cell retention, by e.g. increasing the effective size of the micro-organism 'particles'. Operation of fermentation system thus is dependent on actual production rates, feed concentration, liquid phase mass balances. The product residence time and the (aqueous) hydraulic residence times often are order of magnitude apart. Preferably desired matter does not level with either the product or the aqueous liquid. While undesired solid are removed. This reinforces the importance of being able to control the SRT of the different solid fractions that can occur in a fermentation process.

If desired, the liquid phase comprising the product of interest can be subjected to further downstream processing. This can be done in a manner known per se, e.g. by one or more filtration steps, by back extraction to a lower boiling solvent, and/or solvent or product evaporation as described in WO2021/010822 or in PCT/NL2024/050668, optionally followed by further purification.

### Separation of biocatalytic micro-organism and solid produced organic substance

A method according to the present disclosure is particularly suitable to separate cells of the micro-organism used to fermentatively produce an organic substance from solid matter comprising the produced organic substance. Such organic substance can be a substance that is liquid under the fermentation conditions or extracted from the reaction mixture by in situ extraction in the fermentation zone, yet subjected to solidification prior to separation in the separation zone (in particular by reducing the temperature to a temperature below the solidification temperature). The produced organic substance can also be an organic substance that is solid both under fermentation conditions and separation conditions.

A substance can be present as a solid phase if present in a concentration is above the solubility of the substance in the aqueous broth (or the solubility of the substance in an 2nd liquid phase). A substance is solubilized if the concentration is within the solubility limits of the liquid phase(s).

When the size of (particles of) the product (containing or consisting of the fermentatively produced organic substance of interest) is sufficiently smaller than the size of the biocatalytic micro-organism, it is usually possible to use flotation. The upward movement of the solid product particles can be stimulated with gas bubbles, and the product can then be recovered at the gas-liquid interface on top of the liquid in the separation zone, or by catching the product at another interface. I.e. solid is often preferentially present at the interface and can thus move upwards or downwards with a dispersed phase, being gas bubble, liquid droplet or solid particulate, depending on motion of the dispersed phase.

Selective solid-solid separation is particularly beneficial in case of product and/or biocatalyst degradation over time as result of product stability by itself or as result of operational conditions (temperature, pH, shear force/power input, gradients within the reactor system, flow regime) or compounds (e.g. enzymes, biocatalytic species, oxygen, ionic/reactive species) present in the biocatalytic aqueous liquid phase.

In an embodiment comprising the separation of biocatalytic micro-organism and solid produced organic substance, preferably the solid matter for the second fraction comprises at least one organic substance (at least solid under separation conditions) selected from the group of proteins, fats, long chain alcohols, (typically C10 or more, preferably C12-C24), solid flavour molecules, vitamins, pigments and dyes. In particular, good results are achieved with a protein that is fermentatively produced and separated from the micro-organism. Such protein is advantageously selected from dairy proteins, meat proteins, egg proteins and enzymes. As the micro-organism used for the fermentative production, usually a genetically modified micro-organism is used, wherein the genetic modification allows the micro-organism to produce and excrete the protein. In principle any micro-organism suitable for fermentative production of the organic substance of interest can be used. In particular, a micro-organism can be used as described elsewhere in the present disclosure or in the prior art cited herein.

In a highly advantageous embodiment the micro-organism used for the production of the organic substance, preferably protein, that is separated as a solid from the micro-organism, is a fungus, preferably a yeast, more preferably *Pichia; Aspergillus Niger* or *Trichoderma Reesei*; the first fraction enriched in biomass is enriched in the yeast cells and the second fraction is enriched in the produced organic substance, preferably protein; the separation into said first fraction and said second fraction comprises the formation of aggregates comprising the fungus, and allowing said aggregates to settle in the separation zone, thereby forming the first fraction, enriched in the fungus cells, in a lower liquid layer in the separation zone and the second fraction enriched in the produced organic substance, preferably protein, in an upper layer. Selective settling of the fungus aggregates can further be enhanced by choosing a favourable flow channel geometry in the separation zone in combination with a selected liquid velocity (low Re), suppressing upward movement of the yeast aggregates. The inventors realised that principles described for upward movement of liquid recovery phase, containing produced organic substance, in WO2021/010822 or WO2024/049295, can also be translated not only to upward movement of droplets of low density, but also to downward movement of solid particles, such as yeast aggregates more effectively. In particular, the configuration where separation zone is followed after the downcomer (WO2021/01822), with suitable removal of gas phase, or lateral flow into a separation section (WO2024/049295), can be applied in combination with the present disclosure to enhance the retention possibilities of the micro-organism with which the first fraction is enriched.

### Separation of biocatalytic micro-organism and other solid biomass

The inventors further realised that it would be desirable to provide a method for removal of biomass material with reduced to no activity from the bioreactor system, whilst retaining active biocatalyst. Inventors found that by selectively removing biomass material with reduced to no activity the decline in biocatalytic activity over time in the fermentation zone is reduced, preferably to the point where substrate conversion rates can be maintained for prolonged run duration. This can advantageously provide one or more of benefits, like improved yield, reduced downtime, reduced resource requirements, reduced energy consumption, all leading to improved sustainability and improved economic feasibility of the process.

Thus, in a further advantageous embodiment a method according to the present disclosure comprises a separation wherein the first fraction is enriched in the living micro-organism, having biocatalytic activity in the production of the microbiologically produced organic substance, and the second fraction is enriched in microbial material with reduced to no biocatalytic activity in the production of the microbiologically produced organic substance. Typically, said microbial material with which the second fraction is enriched comprises at least one matter selected from the group of cell debris, lysed cells, protein (cellular protein natural to the micro-organism, protein produced as the organic substance of interest or both), and the like. The first fraction or a substantial part thereof, including living micro-organism is returned to the fermentation zone, whilst second fraction can be discarded or taken from the bioreactor system and subjected to downstream processing, e.g. comprising recovery of the produced organic substance of interest if this is present in the second fraction. Hereby, the content of biocatalytically active micro-organism is selectively retained relative to biocatalyst material with no or reduced biocatalytic activity, and a high concentration of the biocatalytically active micro-organism can be maintained in the fermentation zone for a prolonged time.

In particular, good results with respect to the separation of living micro-organism and further microbial mass are achieved with an auxiliary liquid for which the microbial material (such as one or more of cell debris, lysed cells, cellular protein, fermentatively produced protein of interest) has an affinity. The auxiliary liquid is a liquid that forms a separate phase from the aqueous phase. Preferably the density of the auxiliary liquid is lower than the density of the aqueous phase, and auxiliary phase forms a layer on top of the aqueous phase in the separation zone. Such method advantageously comprises dispersing the auxiliary liquid into the reaction mixture or the aqueous phase of the reaction mixture, allowing the said microbial biomass to be enriched at the interface of dispersed auxiliary liquid and the aqueous phase, wherein in the separation zone the auxiliary liquid is allowed to form an upper liquid layer comprising the second fraction, enriched in microbial biomass having reduced to no biocatalytic activity in the production of the microbiologically produced organic substance, and a lower liquid layer, comprising the first fraction, enriched in the living organism having biocatalytic activity in the production of the microbiologically produced organic substance. The auxiliary liquid as a whole, enriched in the further solid biomass, may be taken as the second fraction, or - when in particular the interface between aqueous phase and auxiliary liquid phase is enriched - the volume close to the interface may be taken as the first fraction.

It should be noted that at least in some embodiments, the biomass may potentially act as an emulsion stabiliser for the auxiliary liquid. In such case, similar to emulsion stabilization of dispersed droplets in aqueous system, where emulsion is avoided by control of the coalescence and break-up of the droplets (e.g. having a net outcome of a droplet diameter >20micron.), tailoring the biomass levels and specific interaction at the liquid-liquid interface is required. In the art surfactants, aggregating chemicals, ionic concentrations, temperature are examples known to impact this stabilization.

Suitable auxiliary liquids include liquids that have interactions with the other solids, allowing enrichment of these solids into or on the interface of the auxiliary liquid and are dependent on the nature of the cell debris or protein or biomass. Preferred are liquids that do not mix with water (are at least substantially insoluble) and that are non-toxic to the product producing micro-organism. Preferred is a net repulsive interaction of the cell wall of the active microbial cells (or aggregates/filaments) with the auxiliary liquid's liquid-surface.

Advantageously, the first fraction contains solid particles formed of a plurality of the living micro-organism (clusters), which particles have a net downward impulse (orientation with the gravity force vector). The difference in the physical property, more specifically size, is enhanced by forming enlarged biomass particulates from the micro-organism used for the microbial conversion, e.g. by creating clusters of micro-organism cells, which forming comprises at least one of aggregation, flocculation, and filamentation, and wherein said first fraction comprises said enlarged biomass particulates. This leads to being able, depending on specific solid matter property to apply sedimentation, settling and/or flotation.

In an embodiment, the produced organic substance of interest has a higher affinity for the auxiliary liquid than for the aqueous phase. In such case, at least in some embodiment, the auxiliary liquid may also be suitable as a product recovery phase, by using an in situ fermentative extraction, essentially as described in WO2021/010822 or WO2024/049295. In such case at least the bulk of the auxiliary liquid (remote from the interface with the aqueous phase) may be recovered as a third fraction. The auxiliary liquid does not have to extract the produced organic substance into the bulk of its phase in order to be useful in the product recovery. One may also make use of a hydrophobic interaction wherein the produced organic substance is enriched at/near the interface of the auxiliary liquid phase and the aqueous phase (forming the first fraction).

In an embodiment the auxiliary liquid is also used to enrich produced protein and allow more enriched recovery, where the auxiliary liquid forms an aqueous two phase system with the aqueous phase of the reaction mixture (such as an emulsion of the water-in-water type).

### Separation of specific biocatalyst cells from other cells or microorganisms

Additionally, the inventors realised that a method according to the present disclosure can be used to selectively separate the active micro-organism (as part of the first fraction), of which it is desired to return it to the fermentation zone and use it again for the fermentative production of interest from a different micro-organism (as part of the second fraction).

This embodiment is amongst others particularly advantageous for slow growing, low biomass yield processes and/or processes with initial growth phase, followed by production phase with less /no growth and higher product yield.

Further, the method of the present disclosure may, also be used advantageously for a specific microbial population enrichment in the system by using a difference in microbial (cell) size, a difference in cell membrane or a difference in density as basis for separation. This is particular beneficial in case of a contamination that builds up over time, which in particular slow/not growing micro-organisms are sensitive to. Typically a run needs to be terminated when the contamination build up reaches a certain level. By selectively removing the contaminating species a longer run duration and desired product formation can be maintained for longer periods of time.

The different micro-organism can be of a different species (typically a contamination), a micro-organism of a different variety of the same species, or micro-organism of the same variety having a different phenotype. The different micro-organism (with which the second fraction is enriched) generally has an undesired or less desired property, such as a reduced activity or lack of activity with respect to the fermentative production of the organic substance of interest, compared to the desired active micro-organism.

Separation into the first fraction and second fraction can generally be based on one or more of the principles described above; e.g. use can be made, for example, of a difference in size, resulting in a difference in gravitational, buoyancy and/or drag forces, allowing for selective separation by settling Other physical properties that influence selective solid separation: specific gravity, electrostatic forces, polarity/non-polarity, size, magnetic, hydrophobicity.

### Separation of biocatalytic cells form inorganics, salt particles, side-products and the like

In a further embodiment, the second phase is enriched in an inorganic solid, salt particle, solid side-product or the like. This can also be accomplished using a technique of which the principle is described above, e.g. by selectively forming clusters of either the biocatalytic micro-organism or the solid matter that is to be separated from said micro-organism, thereby increasing the size of one of the solid matters relative to the other, and carrying separation on the basis of a difference in size.

### Micro-organism used for fermentation

Advantageously, a micro-organism is used for the fermentative production of the organic substance of interest selected from the group of bacteria, archaea and fungi, preferably selected from the genera *Pseudomonas, Gluconobacter, Rhodobacter, Clostridium, Escherichia, Paracoccus, Methanococcus, Methanobacterium, Methanocaldococcus , Methanosarcina, Aspergillus, Penicillium, Saccharomyces, Kluyveromyces, Pichia, Komagataella, Candida, Hansenula, Bacillus, Corynebacterium, Blakeslea, Phaffia (Xanthophyllomyces), Yarrowia, Schizosaccharomyces, Zygosaccharomyces, Saccharopolyspora, Trichoderma* and *Zymomonas* more preferably from the group of *Corynebacterium glutamicum, Escherichia coli, Bacillus subtilis, Bacillus methanolicus, Pseudomonas aeruginosa, Pseudomonas putida, Rhodobacter capsulatus, Rhodobacter sphaeroides, Paracoccus carotinifaciens, Paracoccus zeaxanthinifaciens, Saccharomyces cerevisiae, Saccharomyces pastorianus, Schizosaccharomyces pombe, Aspergillus nidulans, Aspergillus niger, Aspergillus oryzae, Blakeslea trispora, Penicillium chrysogenum, Phaffia rhodozyma (Xanthophyllomyces dendrorhous), Pichia pastoris (also known as Komagataella phaffii), Yarrowia lipolytica, Saccharopolyspora spinosa, Trichoderma reesii and Zymomonas mobilis,* in particular from the group of *Escherichia coli, Bacillus subtilis, Pseudomonas aeruginosa, Pseudomonasputida, Saccharomyces cerevisiae, Saccharopolyspora spinosa, Trichoderma reesii, Pichia pastoris (Komagataella phaffii), Aspergillus niger and Zymomonas mobilis.*

A micro-organism selected from the above micro-organisms may in particular be used for the production of an organic substance disclosed herein. The skilled person will be able to a choose particularly useful micro-organism for the fermentative production of a specific organic substance of interest, based on the present disclosure and methodology known in the art.

### Fermentatively produced organic substances of interest

A method according to the present disclosure can be used in the production of any organic substance that can be produced fermentatively. The produced organic can be solid, it can be dissolved in the liquid phase and/or a second liquid phase (auxiliary liquid or product recovery phase) or it can form its own phase. This product own phase can also be used for selective solid removal combining both product and second solid fraction removal.

In a method wherein a microbiologically produced organic substance is separated from the first fraction as part of the second fraction (see also above), it can form a solid phase at least in the conditions used in the separation zone, in particular at least at a temperature of about 40 degrees C or less, preferably at a temperature in the range of between 0 degrees C and 35 degrees C, more preferably at a temperature in the range of between 4 and 30 degrees C.

The fermentatively produced organic substance is excreted by the micro-organism, such that it does not require lysis of the micro-organism.

Typical process applications of a method according to the present disclosure include the production of biofuels / biofuel precursors, chemicals production, like biobased replacements/precursors for petrochemicals such as polymers for plastics; agricultural chemicals such as fertilizers, pesticides and herbicides, speciality chemicals and pharmaceuticals and food (additives), including proteins, amino acids, vitamins, microbial oils, flavours.

Usually, the organic substance has at least 2 carbons, preferably at least 6 carbons, in particular at least 10 carbons, more in particular at least 12 carbons. The organic substance can have over a 1000 carbons (such as in case of produced biopolymers. In an embodiment the organic substance has up to 500, up to 250, up to 100, up to 50, or up to 24 carbons.

Advantageously, the method according to the invention is used to produce one or more organic substances selected from the group consisting of hydrocarbons, in particular monoterpenes, sesquiterpenes, aromatic hydrocarbons; isoprenoids (terpenoids); organic acids, in particular C5-C24 fatty acids, more in particular C12-C20 fatty acids; alcohols, in particular alcohols having at least 4 carbon atoms (in particular butanol or long chain alcohols); phenolic compounds, ketones, in particular ketones having at least 5 carbon atoms; aldehydes in particular aldehydes having at least 5 carbon atoms; cyclic carboxylic esters, in particular lactones; non-cyclic esters, in particular non-cyclic esters having at least 5 carbon atoms; lipids in particular glycerides; ethers; pyridines; imines; imides; amines; amino acids; and peptides. For further details of preferred organic substances that can in be produced in accordance with the invention, reference is made to the prior art cited herein, in particular WO2021/010822 page 23, line 27 till page 27, line 10 of which the contents are incorporated herein by reference.

Further particularly preferred is a method of the present disclosure wherein a protein is fermentatively produced as the organic substance of interest. Usually, the protein is produced by a genetically modified micro-organism, wherein one or more genes have been inserted or modified to allow expression of the protein (if the protein is not naturally produced by the wild-type micro-organism), to enhance expression, and/or to allow or enhance excretion by the micro-organism. The protein may e.g. serve as a food ingredient, a feed ingredient or pharmaceutical.

The method of the present disclosure is in particular advantageous for the production of a dairy protein, such as at least one of Lactoferrin, Casein, Lactoglobulin, Lactalbumin; a meat protein, such as at least one of collagen-like/collagen, leghemoglobin, myoglobin; and egg protein, such as at least one of Ovomucoid and Ovalbumin.

Modifying a micro-organism to make it suitable for the production of a protein can be done based on methodology generally known in the art. Further, specific means of modification are e.g. described in Domingues et al (Process Biochemistry , March 2005, Pages 1151-1154; https://doi.org/10.1016/j.procbio.2004.04.016), describing *Aspergillus niger* β-galactosidase production; Robert et al (Biochemical Engineering Journal, Volume 147, 15 July 2019, Pages 39-47; https://doi.org/10.1016/j.bej.2019.03.027) describing recombinant lipase B in *Pichia pastoris;* Aro et al (Food Research International Volume 163, January 2023, 11213; https://doi.org/10.1016/j.foodres.2022.112131) describing bovine beta-lactoglobulin and hen egg ovalbumin by *T reesei ;* Shao et al. (Bioresource Technology, Volume 363, November 2022, 127884; https://doi.org/10.1016/j.biortech.2022.127884) describing leghemoglobin production in *P. pastoris.*

When producing a protein, it is particularly preferred to use the method of the present disclosure to separate the produced protein as part of the second fraction from the first fraction. Separating the produced product from the reaction mixture already in the bioreactor system in the separation zone (semi-) continuously, instead of further downstream, allows separation at an early stage from components in the reaction mixture that may degrade or otherwise modify the protein. E.g. fermentation broths often contain proteases. The method according to the invention makes it possible to produce proteins effectively whilst having relatively low retention times in the reaction mixture, which addresses product instability problems. Low retention times also allows operation at a higher substrate concentration (less dilution), which increases production capacity, yield, and offers the possibility to make downstream processing easier.

For the fermentative production of a protein in accordance with the present disclosure one may use bacteria. Gram negative bacteria are not preferred for food applications, due to the presence of endotoxins. Benefits of using bacteria are: fast growth, low complexity media, a broad product titre range. Examples of suitable bacteria include : *E.coli* and *C. glutamicum.*
Good results are obtained in particular with a fungus, such as a yeast. Yeast species do have the eukaryotic post translation modifications (PTMs) to produce the proteins. Usually they offer by a medium titer. 1-22 g/L. Preferred examples are :, *S. cerevisiae, K.marxianus* and *Pichia pastoris (K. phaffi).* Further, Filamentous fungi are particularly useful for the fermentative production of a protein or other organic substance in accordance with the present disclosure. Their filamentation can be used to facilitate separation in order to obtain the first and second fraction. They offer a high biomass density, high titers, eukaryotic PTMs, longer effective fermentation times. For filamentous based processes, *Trichoderma,* in particular *T. reset,* and *Aspergillus,* in particular , *A. niger,* are preferred filamentous fungi.

## Claims

1. A method for selective cell retention of a micro-organism during a continuous or semi-continuous fermentative production of one or more organic substances in a bioreactor system, the bioreactor system comprising a fermentation zone, and a separation zone, which fermentation zone and separation zone are fluidly connected, the method comprising
- providing a reaction mixture in the fermentation zone, which reaction mixture comprises the micro-organism and an aqueous phase, the aqueous phase comprising a substrate for the micro-organism;
the method comprising a stage during which the fermentative production and a separation are simultaneously carried out, wherein
- at least during a substantial part of said simultaneous production and separation stage, substrate for the micro-organism is continuously or semi-continuously fed into the bioreactor;
- at least during a substantial part of said simultaneous production and separation stage, the reaction mixture, comprising the micro-organism and the produced organic substance, or at least an aqueous fraction of the reaction mixture, is continuously or semi-continuously fed from the fermentation zone into the separation zone,
- subjecting the reaction mixture, comprising the micro-organism and the produced organic substance, or at least an aqueous fraction of the reaction mixture, fed into the separation zone, to a separation into at least two fractions, thereby forming a first fraction enriched in the micro-organism and a second fraction enriched in solid matter different from said micro-organism in which the first fraction is enriched, wherein the separation into said first fraction and said second fraction makes use of at least one difference in a physical property between said micro-organism in the first fraction and said solid matter in the second fraction, which physical property is selected from the group of specific gravity, electrostatic forces, polarity/non-polarity, size, shape, magnetic properties and hydrophobicity;
- at least during a substantial part of said simultaneous production and separation stage, at least a part of said second fraction is removed continuously or semi-continuously from the bioreactor system;
- at least during a substantial part of said simultaneous production and separation stage, at least part of the first fraction, enriched in the micro-organism, is continuously or semi-continuously fed from the separation zone back into the fermentation zone.

2. The method according to claim 1, wherein said first fraction enriched in said micro-organism is retained in the bioreactor system by alignment of internal flow hydrodynamics, system geometry and effective micro-organism 'particle' size, wherein
said internal flow hydrodynamics are turbulent conditions (preferably a Re of more than 3000) in the fermentation zone and less turbulent (preferably Re of 2900 or less), essentially laminar conditions (Re < 2300), in the separation zone,
wherein the solid matter different from said micro-organism, comprises one or more of the following: cell debris, lysed cells, protein, and the like, solid phase product, extracellular matter, different aggregation and/or flocculation state of the micro-organism(s) from said micro-organism in the first fraction, wherein said solid matter is separated from the first fraction by aqueous phase liquid withdrawal from the separation zone, and/or presence of a second liquid phase in the system, and/or presence of a gas phase in the separation zone.

3. The method according to claim 1 or 2, wherein the separation into said first and said second fraction is aided by the presence of an organic second liquid phase, said second liquid phase comprising either a (by)product forming its own liquid phase and/or auxiliary liquid phase supplied to the bioreactor system.

4. The method according to any of the preceding claims, wherein the separation into said first and said second fraction is aided by the presence of an aqueous second liquid phase, said second liquid phase comprising an aqueous phase, containing one or more polymers and optionally a salt.

5. The method according to any of the preceding claims, wherein the separation into said first and said second fraction is aided by the presence of a gas phase, said gas phase either being locally introduced and/or being the result of gas entraining in the aqueous fraction to the separation zone as result of hydrodynamics.

6. The method according to any of the preceding claims, wherein
- the retention time of said first fraction is significantly larger than the hydraulic retention time of the (main) aqueous phase in the bioreactor system;
- the retention time of said second fraction is equally or lower than the hydraulic retention time of the (main) aqueous phase in the bioreactor system; and
- in case the separation is aided by the presence of a second liquid and/or gas phase the retention time of said second fraction is significantly lower than the hydraulic retention time of the (main) aqueous phase in the bioreactor system.

7. The method according any of the preceding claims, wherein the solid matter by which said second fraction is enriched comprises the microbially produced organic substance, preferably a substance selected from the group consisting of proteins, fats, long chain alcohol, solid flavour molecules, vitamins, pigments and solid dyes.

8. The method according to claim 7, wherein the microbially produced organic substance comprises a protein, preferably a protein selected from dairy proteins, meat proteins, egg proteins and enzymes.

9. The method according to claim 7 or 8, wherein
the micro-organism used for the production of the organic substance, preferably protein, comprises *Pichia* cells;
the first fraction enriched in biomass is enriched in the *Pichia* cells and the second fraction is enriched in the produced organic substance, preferably protein;
the separation into said first fraction and said second fraction comprises the formation of aggregates comprising *Pichia* cells and allowing said aggregates to settle in the separation zone, thereby forming the first fraction, enriched in the *Pichia* cells in a lower liquid layer in the separation zone and the second fraction enriched in the produced organic substance, preferably protein, in an upper layer.

10. The method according to any of the preceding claims, wherein the first fraction comprises particles of micro-organism cells of the micro-organism with which the first fraction is enriched, such as aggregates, flocs, filamented micro-organism, said particles having an observed settling rate of 0.002 cm/min to 1.4 cm/min (typically corresponding to a 'spherical' particle size of about 7 micron to about 0.5 mm).

11. The method according to claim 10, wherein said particles have an observed settling rate of 0.01 cm/min to 0.8 cm/min (typically corresponding to a size of about 10 micron to about 0.3 mm).

12. The method according to claim 11, wherein said particles have an observed settling rate of 0.08 cm/min to 0.5 cm/min (typically corresponding to a size of about 50 micron to about 0.15 mm).

13. The method according to any of the preceding claims, wherein said simultaneous production and separation stage comprises an essentially steady state.

14. The method according to claim 13, wherein during said essentially steady state substrate is fed from the fermentation zone into the separation zone, said separation into said first fraction and said second fraction is carried out, at least a part of said second fraction is removed from the bioreactor system, and at least part of the first fraction, enriched in the micro-organism, is fed from the separation zone back into the fermentation zone.
